# EUROPEAN PATENT APPLICATION

(11) **EP 2 113 260 A1**
(43) Date of publication of application: **04.11.2009**
(21) Application number: 09158913.5
(22) Date of filing: 28.04.2009
(51) Int. Cl.: A61L 27/04, A61L 27/30

(54) **Implant surfaces and treatments for wear reduction**

(30) Priority: 28.04.2008 US 110404
(71) Applicant: DePuy Products, Inc., Warsaw, IN 46581 (US)
(72) Inventor: Yang, Sophie Ziaofan, Warsaw, IN 46580 (US); Salvati, Lawrence Jr, Goshen, IN 46526 (US)
(74) Representative: Belcher, Simon James

(57) **Abstract**

A method of treating an implant to reduce wear characteristics comprises forming a plurality of pits on at least a portion of a chromium-cobalt alloy surface, the pitted surface having reduced inclusion content relative to an unpitted chromium-cobalt surface. At least a portion of the pitted surface is then smoothed. A corrosion resistant oxide-containing layer is created on the smoothed pitted surface. The layer is modified by selectively enhancing the relative amount of a component in the oxide-containing layer.

## Description

The invention relates to surface treatment of surfaces, for example to help to reduce wear.

Implants for use as replacement structures in patients suffering from, for example, joint degradation, have become widespread in their application. Improving the durability and longevity of such implants has numerous advantages such as extending the lifetime of such implants, and reducing the need for future replacement of such implants.

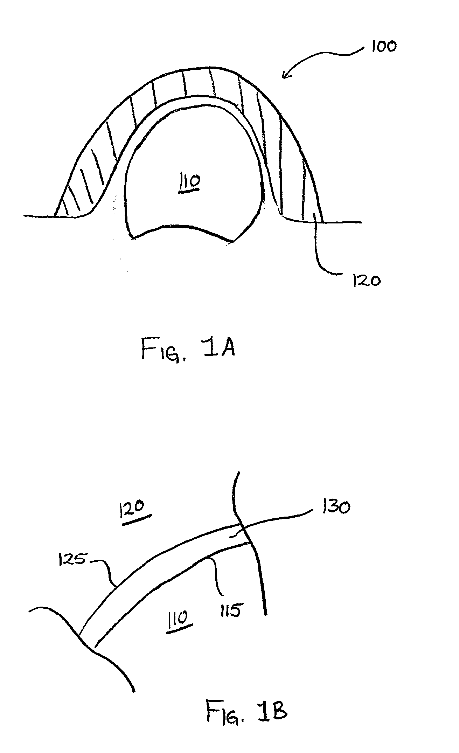

FIGS. 1A and 1B depict a portion of a head-cup joint implant 100. The head 110 and the cup 120 of the implant can move relative to one another as a ball in socket joint. In instances where the head 110 and cup 120 are each metallic, the opposite surfaces 115, 125 of the structures 110, 120, which can be complementary in shape, can be termed metal-on-metal (referred to as "MOM") surfaces, which form an interface 130 between the structures 110, 120. As depicted in FIGS. 1A and 1B, a MOM surface does not always contact its complementary surface. As shown in the blown up schematic of FIG. 1B, opposite MOM surfaces 115, 125 can be configured to be separated by a given distance, which can be filled with an interstitial bodily fluid upon implantation to allow relative movement between the surfaces 115, 125. Occasional contact between MOM surfaces, however, is not necessarily prohibited.

It can be desirable to manufacture MOM surfaces to have one or more low wear characteristics, e.g., the surfaces tending to not emit particular matter and/or having a relatively smooth surface. For example, it can be advantageous to prevent the build up of particles in the interfacial region between MOM surfaces. Such particles can grind and degrade a MOM surface, leading to increased friction between joint surfaces that further accelerates degradation of the implant surface. Accordingly, a need exists for improved implants with surfaces that tend to have one or more low wear characteristics. As well, a need exists for processes to create implants with such surfaces, such as modified MOM surfaces.

The present invention provides methods of treating an implant which can result in one or more reduced wear characteristics upon use of the implant in a subject. Treated implants can include metallic-based implants such as a chromium-cobalt alloy containing implant, and/or a metal-on-substrate surface of an implant. The reduction in wear can be relative to an implant surface that does not receive the treatment. A plurality of pits can be formed in at least a portion of an implant. Pit formation can include using a chemically-based etching technique, such as an electrochemical-based etching technique. Pit formation can optionally be the result of the removal of a plurality of inclusions from the implant surface. The pitted surface can have an oxide-containing layer thereon, which can increase the corrosion resistance of the pitted surface relative to not having the oxide-containing layer present. The oxide-containing layer can be formed by treating the surface with a plasma having one or more oxidative components. In some embodiments, the oxide-containing layer can be highly corrosion resistant, and/or have a high Cr/Co ratio, and/or be thick (e.g., thicker than what would naturally occur for a given alloy when exposed to a selected environment such as ambient conditions). In some instances the oxide-containing layer can have a chromium-to-cobalt ratio greater than about 2 or in a range from about 2 to about 20, and/or a thickness greater than about 10 Å.

Accordingly, in one aspect, the invention provides a method of treating an implant to reduce wear characteristics when implanted in a patient, comprising:
forming a plurality of pits in at least a portion of a surface of the implant; and
forming a highly corrosion resistant oxide-containing layer on the pitted surface, the oxide-containing layer increasing the corrosion resistance of the pitted surface relative to a naturally-occurring oxide layer on the surface.

In another aspect, the invention provides an implant component having a bearing surface of a metal-on-substrate interface, the surface having a plurality of pits, and comprising an oxide-containing layer having a chromium-to-cobalt ratio greater than about 2, a thickness of the oxide-containing layer being greater than about 20 Å.

The implant can be designed so that, when it is implanted, the bearing surface is substantially free from contact with a bodily fluid.

Preferably, a portion of the bearing surface has a surface roughness characterized by a Ra value less than about 0.6 µm.

Preferably, the chromium-to-cobalt ratio is less than about 20.

Preferably, the bearing surface has a reduced population of inclusions having a size smaller than about 15 µm.

Preferably, the bearing surface has a reduced population of inclusions relative to an untreated cobalt-chromium surface.

Preferably, the density of pits on the bearing surface is at least about 10 pits/100 µm².

Preferably, the component is formed from a chromium-cobalt alloy.

In a further aspect, the invention provides a method of treating an implant to reduce wear characteristics, comprising:
forming a plurality of pits on at least a portion of a chromium-cobalt alloy surface, the pitted surface having reduced inclusion content relative to an unpitted chromium-cobalt surface;
smoothing at least a portion of the pitted surface;
creating a highly corrosion resistant oxide-containing layer on the smoothed pitted surface; and
modifying the oxide-containing layer by selectively enhancing a relative amount of at least one component in the oxide-containing layer.

The implant surface can be contacted with an acid during surface treatment. In some embodiments, the acid is contacted with the implant surface after the oxide-containing layer is formed. The latter can aid in selectively enhancing a relative amount of one or more components in the oxide-containing layer, though other techniques can also be potentially utilized. For example, the acid can be used to treat an oxide-containing layer, resulting in an enhanced chromium-to-cobalt atomic ratio (e.g., greater than about 2) by selectively removing cobalt oxide relative to chromium oxide. In some embodiments, an implant surface can be smoothed before the oxide-containing layer, which can be thick, is formed thereon, and optionally after the pits are formed on the surface. The smoothing can be carried out by any number of processes such as polishing. The smoothing can result in at least a portion of the implant surface having a smoothness characterized by a Ra value less than some designated value such as about 1 µm, about 0.6 µm, or 0.1 µm.

Some exemplary embodiments are directed to implants having a surface with reduced susceptibility to wear. The implant, which can include a metallic substrate such as a chromium-cobalt alloy, can be a man-made product, which is free from contact with a bodily fluid. The implant can have a substrate which can be adapted to include a metal-on-substrate (MOS) surface having a plurality of pits. The pits can be characterized by a pit density greater than some selected value such as 10 pits/100 µm². The MOS surface can also be depleted of inclusions having a size smaller than a selected value such as about 15 µm, and/or relative to an untreated cobalt-chromium surface. The MOS surface can have an oxide-containing layer with a chromium-to-cobalt atomic ratio greater than about 2. The chromium-to-cobalt ratio can optionally be less than about 20, about 15, or about 10. The oxide-containing layer can have a thickness greater than about 20 Å. The MOS surface can have a portion having a surface roughness characterized by a Ra value less than about 1 µm, about 0.6 µm, or about 0.1 µm.

The invention provides devices that can include a substrate surface with one or more low wear characteristics. Accordingly, surfaces of substrates, such as can be used in implants such as prosthetic implants, can have one or more characteristics such as reduced inclusions (e.g., carbides) content, a pitted surface which can be smoothed, and a passivation layer with a composition that resists corrosion and/or wear. In many embodiments, these surfaces are meant to interface with another substrate surface; this can lead to degradation of the surfaces after extended relative motion between the surfaces if one or more low wear characteristics are not imparted to the surface. The term "surface" is used to refer to a boundary of the object, which can include an immediate locality of material adjacent to that boundary. Accordingly, the surface can comprise a thickness of one or more layers of atoms, and can be as thick as about 10 nm.

Though aspects of the low wear characteristic surfaces can be applied to a variety of substrates, several embodiments are directed to substrates having a metallic surface. Such substrates can include a metallic substrate possessing the metallic surface (which can be similar or different in metallic character), or a non-metallic substrate coupled to a metallic layer, coating, or body that has the metallic surface. In general, the term "metallic" is used to describe an object having at least some qualities of bulk metals. Accordingly, metallic surfaces and metallic substrates exhibit at least one property similar to that of bulk metals. For example, non-metallic materials that have trace contaminants of metal impurities can be excluded from a description of metallic materials. Some embodiments are directed to metal surfaces that form a portion of a metal-on-substrate (referred to as "MOS") interface. The term "MOS surface" is used to refer to a surface that is a portion of a MOS interface (e.g., a metallic surface or other type of substrate surface). MOS surfaces can be opposed to another substrate surface, which can cause wear of the metallic surface. MOS surfaces are often times complementary in shape, and often represent a portion of an interface region between complementary MOS surfaces.

Non-limiting examples of MOS interfaces include metal-on-metal (MOM), polymer-on-metal, and ceramic-on-metal combinations. A metallic surface of the substrate can comprise any metal that is suitable for implantation into the human body, i.e., any biocompatible metal. Suitable metals include, but are not limited to, titanium, tantalum, and stainless steel. Preferably, the metallic surface comprises a metal selected from the group consisting of titanium, titanium alloys, tantalum, tantalum alloys, stainless steel, a chromium-containing alloy, a cobalt-containing alloy, cobalt-chromium alloy, and cobalt-chromium-molybdenum alloy. In particular embodiments, a metallic surface of an implant comprises a cobalt-chromium-molybdenum alloy. Particular examples of such alloys include wrought cobalt-28chromium-6molybdenum alloys for surgical implants following ASTM designation F 1537-00, and cobalt-28chromium-6molybdenum alloy castings following ASTM designation F 75-01. Some embodiments are directed to metallic surfaces that include a cobalt-chromium alloy with an enhanced Cr:Co ratio. As well, some surfaces are portions of a manufactured implant that are selected to mimic one or more characteristics of a quasi-steady-state wear surface, which can result in a surface with a low wear characteristic.

Wear on a surface can occur by mechanical and/or chemical mechanisms, which can tend to degrade a surface in some manner such as increasing the roughness of the surface. In terms of mechanical degradation, complementary MOS surfaces can occasionally contact one another, for example when complementary parts become misshapen or insufficient fluid exists between the complementary surfaces to provide lubrication. This can lead to surface deformations and particulate production from mechanical impingement. Wear can also be manifested via chemical mechanisms. For instance, an implant surface can slowly degrade over time from exposure to a bodily fluid via corrosion and other chemical degradation mechanisms. In one example, carbides on a surface of an implant can be susceptible to corrosion by bodily fluids, which can result in carbide particulate build-up in the interfacial region between MOS surfaces. Such particulate material can subsequently degrade the MOS surfaces when the surfaces move relative to one another. Either chemical or mechanical degradation can result in the build-up of particulate material in an interface between MOS surfaces. Accordingly, low wear characteristics of a surface can help to hinder mechanical degradation, chemical degradation, or both.

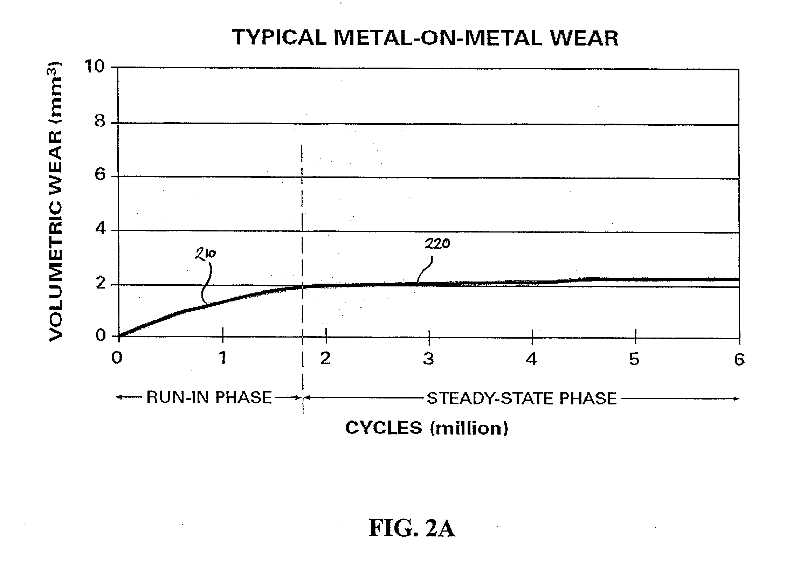

Cyclic wear-testing was conducted on samples of cobalt-chromium-molybdenum (CoCrMo) surfaces to simulate the effect of wear on a MOM surface after implant usage in a subject. FIG. 2A is a graph of data showing the result of metal-on-metal wear at the interface between two CoCrMo surfaces during cyclic testing. The graph depicts two distinct regimes during the wear-testing of the CoCrMo surface. The first regime 210 is a "run-in-regime" during which a substantial amount of metal from the CoCrMo surface is lost. The second regime 220 is a "quasi-steady-state regime" during which the rate of loss of metal from the CoCrMo surface is very low and quasi-steady.

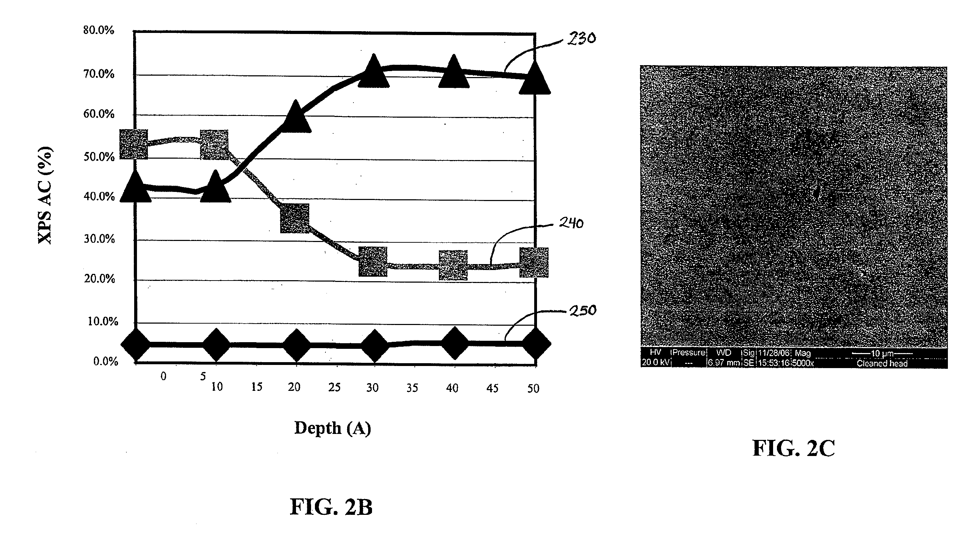

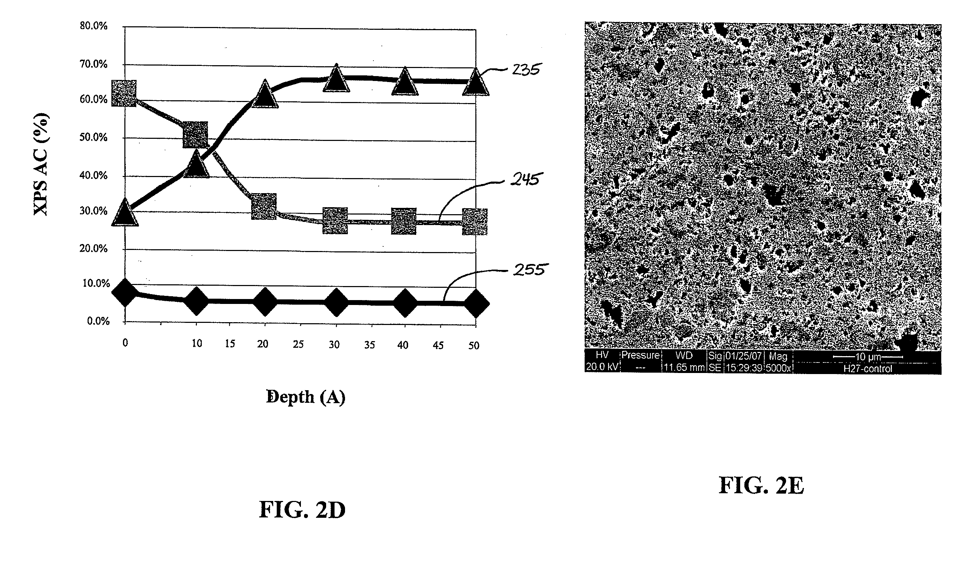

The CoCrMo surfaces at two different times during the wear testing are characterized by FIGS. 2B-2E. FIGS. 2B and 2C characterize a CoCrMo surface before any wear testing is performed. FIG. 2B is a graph of the atomic content of the CoCrMo surface as a function of depth as measured by x-ray photoelectron spectroscopy ("XPS"). The traces 230, 240, 250 indicate the percentages of cobalt, chromium, and molybdenum present as a function of depth, respectively. As indicated by the graph of FIG. 2B, the surface before wear testing has a Cr/Co atomic ratio of about 1.2 at the surface. A scanning electron micrograph ("SEM") of the pre-tested surface, FIG. 2C, indicates that the surface is substantially pit-free and smooth. Further experiments conducted on the pre-tested surface indicate the presence of an oxide-containing layer having a thickness of about 10Å. In contrast, FIGS. 2D and 2E characterize the CoCrMo surface after 3.5 million cycles of wear testing. The traces 235, 245, 255 in the graph of FIG. 2D indicate that after cyclic testing, the quasi-steady-state wear surface has a higher chromium-to-cobalt atomic ratio, about 2, relative to the pre-tested surface. The SEM of FIG. 2E shows that the quasi-steady-state surface has a much higher number of pits as well. Also, additional testing indicates that the surface has a thicker oxide-containing layer, about 15Å thick.

Accordingly, the invention provides methods for preparing surfaces with one or more characteristics of the simulated quasi-steady-state surface from the wear testing. Without necessarily being bound to any particular theory, it is believed that incorporating any of such characteristics in a manufactured surface can help reduce the loss of material during a "run-in-regime" of implantation to help reduce the loss of material, either through chemical mechanisms, mechanical mechanisms, or both. For instance, it is conjectured that a pitted surface can act to store interstitial joint fluid, which can act as a lubricant to help reduce MOS surface contact. As well, thicker oxide layers, or other types of passivation layers, are chemically more resistant to corrosion, particularly when the passivation layer exhibits an elevated chromium to cobalt ratio.

The invention is described by way of example with reference to the accompanying drawings, in which:
FIG. 1A is a side-view schematic of a head-cup joint of an implant;
FIG. 1B is a magnified side view of the interface between the head and cup structures of FIG. 1A;
FIG. 2A is a graph of metal-on-metal wear of a metal-on-metal (MOM) surface as a function of number of cycles tested for a wear test;
FIG. 2B is a graph of atomic composition versus depth for a MOM surface before wear testing was performed on the MOM surface, the atomic composition being determined using x-ray photoelectron spectroscopy (XPS);
FIG. 2C is a scanning electron micrograph (SEM) of the MOM surface before wear testing was performed;
FIG. 2D is a graph of atomic composition versus depth for a MOM surface after 3 million cycles of wear testing were performed on the MOM surface, the atomic composition being determined using XPS;
FIG. 2E is an SEM of the MOM surface after 3 million cycles of wear testing were performed;
FIG. 3 is a flow chart of a method for treating a substrate surface in accord with some embodiments of the present invention;
FIG. 4A is an SEM of the surface of a cobalt-chromium-molybdenum disk, which was chemically etched in 6N HCl, in accord with some embodiments of the present invention;
FIG. 4B is an SEM of the disk surface shown in FIG. 4A after polishing, plasma treatment, and a final HCl treatment;
FIG. 4C is a graph of atomic composition versus depth for the surface of the disk shown in FIG. 4B, the atomic composition being determined using XPS;
FIG. 5A is an SEM of the surface of a cobalt-chromium-molybdenum disk, which was electrochemically etched in 1N HCl, in accord with some embodiments of the present invention;
FIG. 5B is a magnified SEM of a portion of the disk surface shown in FIG. 5A;
FIG. 5C is an SEM of the disk surface shown in FIG. 5A after polishing to a Ra value of about 0.1 µm on an area surrounding the pits;
FIG. 5D is a magnified SEM of a portion of the disk surface shown in FIG. 5C;
FIG. 5E is a graph of atomic composition versus depth for the surface of the disk shown in FIG. 5F, the atomic composition being determined using XPS;
FIG. 5F is an SEM of the disk surface shown in FIG. 5C after treatment by a plasma and HCl;
FIG. 6A is an SEM of the surface of a prosthetic femur head treated with an electrochemical etch, in accord with some embodiments of the present invention;
FIG. 6B is an SEM of the surface shown in FIG. 6A after a polishing treatment; and
FIG. 6C is a graph of atomic composition versus depth for the surface shown in FIG. 6B after the surface is treated with an oxygen plasma and exposed to nitric acid, the atomic composition being determined using XPS.

### Methods of Creating Low Wear Implant Surfaces

The invention provides methods of preparing a surface, which can be used with an implant, to create a low wear surface. Such surfaces can be prepared to incorporate one or more features of a quasi-steady-state surface after wear testing (e.g., a MOS wear-tested surface). Alternatively, or in addition, the surface which has been treated in accordance with the invention can have reduced wear characteristics relative to a surface which has not been treated(e.g., the reduced wear surface can exhibit less wear after a selected number of cycles in a wear test than a surface of a standard metallic substrate such as a ASTM sample). Accordingly, some embodiments can include forming a pitted surface on an implant (e.g., by etching the surface). A passivation layer (e.g., an oxide-containing layer) can be formed on the surface, which can further help reduce degradation of the surface during MOS surface usage, for example.

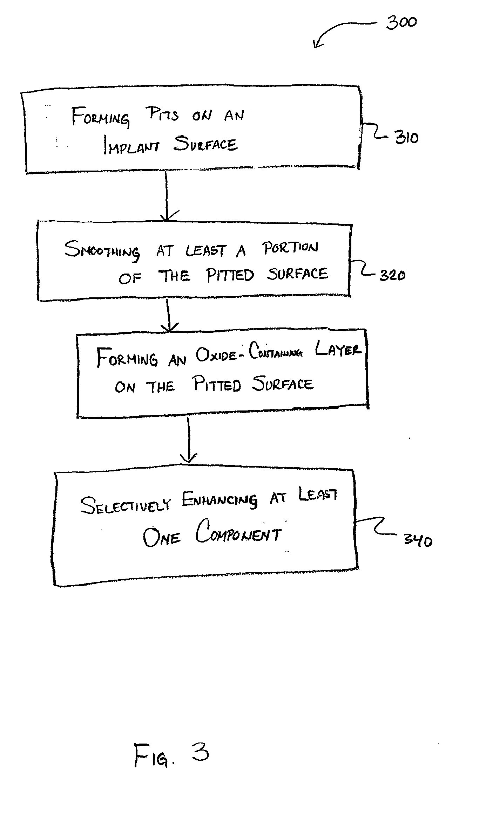

Examples of methods for producing a low wear surface are discussed with reference to the flow chart of FIG. 3. Some methods 300 involve forming a plurality of pits on a surface 310 (e.g., a metallic surface of a portion of an implant). Etching can be utilized to form the pits. The pits can be numerous enough to allow an interface between MOS surfaces to be lubricated by sufficient interstitial fluid stored in the pits. In some embodiments, a substantial number of the pits can be formed by the removal of inclusions such as carbides on the surface and/or in the implant. At least a portion of the pitted surface can be smoothed 320, which can act to help reduce wear between roughened opposed surfaces, for example. An oxide-containing layer can be formed on the pitted surface 330, which can act as a passivation layer. The oxide-containing layer can be selected to have a large enough thickness to impart one or more wear-resistant properties. The oxide-containing layer can be modified to selectively enhance the relative amount of one or more components, e.g., result in an elevated chromium to cobalt ratio, which can result in an oxide-containing layer that can preferentially resist corrosion and/or other wearing action.

Any permutation and combination of the steps in FIG. 3 can be assembled to practice a method consistent with some embodiments of the present invention. Other embodiments can include other steps, or present modification(s) and/or variation(s) to the steps of the method. Each of the steps of FIG. 3 is discussed in further detail in this specification. Any number of the features discussed with respect to each step can be assembled with other steps, or practised individually.

Pits formed in a substrate surface can be characterized in a variety of manners, and can depend upon the nature of the substrate and the size of any inclusions (e.g., carbides). In particular embodiments, the formation is performed in a manner to provide pits in a sufficient number to allow lubrication between opposite surfaces such as opposite MOS surfaces of an implant. For instance, the pit density can be greater than about 10 pits/100 µm². The size of the pits can vary as well. For instance, the pits formed can include an average size which is less than about 15 µm or less than about 10 µm. The average size of a pit can be characterized using any technique understood by those skilled in the art. In one non-limiting example, the average size can be defined by the an average effective diameter calculated using the average cross-sectional area of the pits and assuming a perfectly circular cross section.

In some embodiments, at least some of the pits formed can be from the removal of inclusions, such as carbide inclusions ("carbides"), from the surface of the substrate. Inclusions are particulate matter having a different character (e.g., compositional character) than the surrounding substrate. In some instances, the inclusions are characterized as being susceptible to removal from a substrate when exposed to bodily fluids. Inclusions can include carbide-based particulate material as well as metallic-based particulate material, which can include one or more components such as titanium, aluminum, cerium, and lanthanum. Since some inclusions can be dislodged when exposed to particular bodily fluids, inclusion depletion of a surface (e.g., a metallic surface), before implantation of an implant, can result in advantages such as decreased particulate build-up in the interfacial region of a metallic joint and/or increased resistance to corrosion. Accordingly, a method consistent with some embodiments results in an inclusion-depleted (e.g., carbide-depleted) surface relative to an untreated substrate surface (e.g., the surface of a standardized chromium-containing alloy such as ASTM standard F 1537-00 and/or F 75-01). The level of depletion can vary from partial to substantially absent of inclusions of a particular character. For instance, a surface can be depleted of carbides and/or other inclusions in a selected size range (e.g., less than about 15 µm or less than about 10 µm), or can be substantially depleted of all carbides/inclusions. The size of an inclusion can be defined as the average value of the particulate size of a plurality of inclusions. Such average sizes can be defined using techniques understood by those skilled in the art (e.g., average particle diameter or radius based on a surface area or volume calculation).

The process of forming pits on a surface can occur utilizing any appropriate technique for forming structures consistent with embodiments disclosed herein. For instance, an etching technique can be utilized to form pits and/or remove inclusions from the surface. When etching is performed on a metallic surface, chemical etching techniques can be utilized. In some embodiments, the chemical etching can preferentially result in removal of inclusions (e.g., carbides) to form pits on an exposed surface. It is believed that chemical etching can result in dissolution of the phase boundary between an inclusion (e.g. a carbide particulate) and the implant, which can allow for removal of the inclusion.

Such chemical etching techniques can depend upon the character of the surface to be etched and/or pitted. Chemical etching can be performed utilizing a variety of etching fluids including acid solutions such as an acid including a hydrohalic acid (e.g., HCl), which can optionally include the salt of the acid (e.g., a chloride salt with HCl). For instance, chemical etching of chromium-containing metals (e.g., a cobalt-chromium alloy) can be effectively performed utilizing etching solutions disclosed in US-A-2006/0293758. The concentration, temperature, and time of etching solution exposure can vary depending on what is effective to produce the desired etching result. For instance, for some metallic alloys (e.g., chromium-containing alloys), a hydrohalic acid such as HCl can be used at a concentration of greater than about 3M, at a temperature from about 20°C to about 100°C, at a time for at least about 1 hour.

Chemical etching can optionally include electrochemical etching for certain substrates (e.g., metallic substrates). Electrochemical etching can utilize an appropriate solution with a selected electrical current flowing through the etching solution, while the solution is exposed to the surface to be etched. Electrochemical etching, in some embodiments, can result in more efficient inclusion removal (e.g., more complete inclusion removal versus some purely chemical etching techniques); utilize less harsh chemicals, lower temperatures, and/or shorter etching times relative to some typical chemical etching techniques; and/or result in less total etching relative to some typical chemical etching techniques while still removing inclusions and/or forming pits. Aspects and methods of electrochemical etching that can be used with some embodiments discussed herein are disclosed in US patent application number 12/030689, published as US-A-_.

An electrochemical etching solution can contain at least one electrolyte which transfers current sufficiently to allow electrochemical etching. The etching solution can be an appropriately formulated electrolyte solution such as an aqueous solution with at least one of an inorganic compound soluble in the solution and an organic compound soluble in the solution. Inorganic compounds which can be utilized can be formulated as a solution such as an alkaline solution, an acid solution, a salt solution, or any combination thereof. Alkaline solutions can include any combination of compounds such as hydroxides (e.g., NaOH, KOH) and carbonates (e.g., Na₂CO₃, K₂CO₃, NaHCO₃). Examples of salt solutions include any single type or combination of salts including chlorides, phosphates, sulfates, sulphites, nitrites, nitrates, and mixtures thereof. In a particular embodiment, the inorganic compound includes an acid solution. Examples of acid solutions include any single type or combination of H₂SO₄, HCl, HNO₃, CH₃COOH, H₃PO₄, and HClO₃. Potential organic compounds include sugar and alcohols (e.g., ethanol). The concentration of various components of the solution can be any appropriate for performing electrochemical etching in an effective manner (e.g., the concentration of any component can vary from about 0.01M to the saturation point of the solution, or from about 0.05 M to about 5 M, or can be a relatively mild acid concentration such as below about 5M, 4M, or 3M). The current density applied to the solution can be any appropriate level (e.g., more than about 1 µA.cm⁻²). The process can be applied for a time effective to achieve the desired etching (e.g., a time from about 30 seconds to about 1 hour). The temperature at which electrochemical etching can be performed can be any effective temperature. Relatively low temperatures can be preferred, for example, not more than about 80°C, 70°C, 60°C, 50°C, or 40°C; or in a temperature range around room temperature such as about 10°C to about 35°C.

When a surface (e.g., a pitted surface) is etched, or has some roughness, the surface can be smoothed in some portions consistent with some embodiments of the invention. By smoothing sections of the etched surface, e.g., portions opposite to another surface such as utilized in a MOS interface, the incidence of surface degradation (e.g., roughening by relative motion between MOS surfaces) can be reduced. In such cases, pits in a surface can be maintained while the surface can be smoothed relative to the original surface from etching or other pit formation processes. For instance, the pits can be maintained in a disposition to allow effective lubrication fluid storage for a MOS interface. In some instances, the smoothing of the surface is sufficient such that the Ra value of the etched surface before smoothing is higher than the Ra value after smoothing. The term "Ra" can be defined as the arithmetic average of the absolute values of the profile height deviations from a mean line over some selected evaluated length. In some embodiments, the Ra value of a smoothed surface (e.g., a portion of the surface which is polished and does not include a pit) can be less than about 1 µm, or less than about 0.6 µm, or less than about 0.1 µm.

Techniques which can be used to smooth metallic surfaces (e.g., pitted metallic surfaces) and other surfaces can include polishing. Suitable polishing techniques can include the process of buffing in order to decrease the roughness of the surface. Polishing is a well understood process to those skilled in the art. A variety of grit papers can be used to achieve the desired smoothness for a surface when polishing. For example, some chemically etched surfaces can be smoothed utilizing a 800 grit paper, followed by a 1200 grit paper, using a 0.6 µm and/or a 0.1 µm diamond suspension.. In some electrochemically etched surfaces, a final polishing may be all that is needed to achieve a selected smoothness. The media for polishing can be diamond-based, alumina-based, or any other suitable media.

In some embodiments, the presence of an oxide-containing layer on an implant surface can act to make the surface corrosion resistant and/or reduce the wear rate of the surface (e.g., relative to an implant surface that lacks the oxide-containing layer or has a thin oxide-containing layer). Accordingly, an oxide-containing layer can be a modified oxide-containing layer, e.g., making a thicker oxide layer relative to a naturally occurring oxide layer. In some instances, the oxide-containing layer (e.g., modified) can be highly corrosion resistant, for example to an environment such as a MOS surface exposed to bodily fluids. The high corrosion resistance can be relative to a selected type of surface such as a surface with a naturally-occurring oxide layer or a surface with an oxide-layer subjected to some other type of condition. The oxide-containing layer can be added to a variety of surfaces such as a pitted surface of an implant, an oxide-free surface, and/or a surface having a pre-existing oxide-containing layer to further thicken and/or change the character of the layer. In some embodiments, the oxide-layer can be applied to various types of substrates including metallic surfaces such as chromium-containing surfaces (e.g., a cobalt-chromium-molybdenum alloyed surface).

The term "oxide-containing" is used to refer to a portion including at least one oxide component, though other materials may also be intermingled with the oxide component(s). For example, an oxide-containing film can contain multiple chemical oxides. In various embodiments, the oxide-containing layer can be thick, and/or have a selected thickness. In certain embodiments, the thickness can be larger than the naturally-occurring oxide thickness of the material when subjected a selected environment such as ambient conditions, and/or larger than the oxide thickness of a wear-tested MOS surface after a selected number of cycles (e.g., about 15Å after about 3 million cycles or after about 2 million cycles). In some embodiments, an oxide-containing layer can have a thickness greater than about 10Å, about 15Å, or about 20Å. In some embodiment, the oxide-containing layer has a thickness less than about 200Å, 150Å, or 100Å.

A variety of techniques can be employed to apply a oxide-containing layer. Some embodiments involve exposing a surface to a plasma to form the oxide-containing layer. Aspects and methods of plasma usage which can be employed in connection with the present invention are disclosed in US patent application number 60/956778, entitled "Ultra-Passivation of Chromium-Containing Alloy and Methods of Producing the same", and applications which claim priority from it.

In some embodiments, surfaces are treated with a plasma formed from one or more oxidative components to promote formation of an oxide-containing layer, which can be thick, on the surface. The oxidative component of the plasma can comprise oxygen, water, ethanol, hydrogen peroxide, or mixtures thereof. In some embodiments, the oxidative gas plasma can be formed, for example, from oxygen, a mixture of oxygen with air, or with one of the foregoing along with one or more non-reducible gases. Non-reducible gases include argon, neon, helium, xenon, and krypton. In some embodiments, argon and/or helium are preferred. In some preferred embodiments, the amount of oxygen in the gas is greater than 3%. In some preferred embodiments, the amount of oxygen in the gas is from about 3% to about 100%.

A number of gas plasma treatment systems suitable for use in the practice of aspects of the present invention are commercially available and such systems are understood by those skilled in the art. In some embodiments, a plasma can be generated by creating an electrical discharge in a gaseous atmosphere under suitable pressure conditions. A typical plasma treatment system has a chamber capable of being maintained at a desired pressure (typically atmospheric or below). The surface to be treated is placed within the chamber with the selected gas and an appropriate energy source (e.g., electrical discharge) is used to transform the gas to a plasma. The energy source used in the gas plasma oxidation is one capable of supplying a sufficient amount of energy to ionize at least a portion of oxidative gas to form a gas plasma. At least three power sources have been widely used in practice to supply the energy. These include DC electrical energy, radio frequency (RF) energy, and microwave energy. Since RF energy sources typically have the greatest sensitivity and are the most free from interference, some embodiments utilize a RF energy source.

The conditions under which a plasma can be formed include any that provide a plasma capable of forming an oxide-containing layer consistent with some embodiments disclosed herein. For example, the amount of energy utilized to form the plasma can be from about 100 to about 1000 watts. In some preferred embodiments, the amount of energy is from about 800 to about 1000 watts. In another example, the gas plasma treatment of the surface is can be performed for about 5 to about 120 minutes. In some embodiments, the time is from about 30 to about 90 minutes. In other embodiments, however, the time is at least 60 minutes. In another example, the pressure at which the gas plasma oxidation is performed can be from atmospheric pressure to sub-atmospheric pressure. In some embodiments, the pressure is from about 200 mTorr to about 400 mTorr. In certain embodiments, the pressure is about 300 mTorr. In a further example, during the gas plasma oxidation, the flow rate of the gases through the oxidizing chamber can be from about 100 standard cubic centimetres per minute (sccm) to about 500 sccm. In one preferred embodiment, the flow rate is from about 150 to about 350 sccm ; about 250 sccm is a preferred flow rate in many circumstances.

An oxide-containing layer can be modified to enhance selectively the relative amount of one or more components (e.g., a chromium-oxide component). Such selective enhancement can improve the corrosion-resistance (e.g., improved wear resistance) of a surface by enriching particular component(s) relative to others. For instance, in CoCr alloyed materials, higher Cr:Co ratios can result in improved corrosion resistance relative to materials having a lower Cr:Co ratio. Thus, some embodiments are directed to implant surfaces where the Cr:Co ratio is larger than or equal to the ratio found in wear-tested samples. Accordingly, the Cr:Co ratio can be greater than about 1, 1.2, 1.5, 2, 2.5, or 3. In some embodiments, the Cr:Co ratio is kept below about 20, 15, 10, 9, 8, or 7. The Cr:Co ratio can be determined using a number of techniques such as measuring sputter depth profiles using XPS or other techniques known to those skilled in the art. In some embodiments, the component removed can be an oxide component, a non-oxide component, or a combination of one or more oxide and non-oxide components.

Selective component enhancement can be performed using any technique capable of achieving the compositions consistent with embodiments of the present invention. In some embodiments, the selective enhancement can be performed by treating a surface with an acid (e.g., contacting a plasma-treated oxide-containing layer, which can optionally contain pits, to an acid to remove components from the surface). In some instances, the acid can selectively reduce the presence of one or more components (e.g., removal of one or more oxide components such as Co-oxide relative to one or more other components such as Cr-oxide) to result in a treated surface having an enhanced level of one or more components (e.g., elevated Cr:Co ratio relative to before treatment). In some embodiments, the acid can include nitric acid (HNO₃), hydrochloric acid (HCl), sulfuric acid (H₂SO₄) or citric acid. In some embodiments, the acid includes nitric acid and HCl. The surface can be contacted with a liquid acid at a concentration that suitably passivates the surface. One particular concentration range of the acid is about 2M to about 8M. In certain embodiments, the acid concentration is about 20 to about 30 weight percent 3M to 6M. The surface can be treated for a time sufficient to develop a thick robust Cr rich surface layer. In some embodiments, the surface is contacted with the acid for about 20 to about 120 minutes (30 to 60 min in some embodiments, or about 60 min in other embodiments). The treatment can occur at a temperature of 10°C to 90°C. In certain embodiments, the preferred temperature is about 20°C to about 60°C.

While acid treatment can be used to enhance one or more components selectively in a substrate, it should be understood that acid treatments can generally be used with certain embodiments of the invention, and can be utilized multiple times. An acid treatment can be conducted after selective component enhancement (e.g., plasma oxidation) or conducted before and after selective component enhancement. As used herein, the term "before" includes intervening events that may occur (for example, "before" need not be "immediately before"). Certain embodiments have more than one acid treatment step after selective oxide removal. For example, an acid treatment step using an acid comprising hydrochloric acid followed by another acid treatment step using nitric acid can be utilized.

### Implants with Reduced Wear Surface Properties

The invention provides substrate surfaces which exhibit one or more low wear properties, in which the surface has one or more characteristics that tend to exhibit reduced wear when used in a desired manner. As mentioned herein, substrate surfaces such as metallic surfaces, for example provided any of the metals/alloys mentioned in this document, are often used to provide a portion of an implant which can be inserted into a patient. In some embodiments, an implant can be used as a prosthesis for any suitable part of the body, e.g., a component of a joint in the body. Accordingly, a device according to the invention can be used as a component of an orthopaedic prosthesis, such as, for example, a component of an orthopaedic hip joint prosthesis, a component of an orthopaedic knee joint prosthesis, a component of an orthopaedic shoulder joint prosthesis, a component of an orthopaedic elbow joint prosthesis, a component of an orthopaedic ankle joint prosthesis, a component of an orthopaedic finger joint prosthesis, a component of an orthopaedic spine disk implant, a component of an orthopaedic temporo-mandibular joint (jaw) implant, and others. In many embodiments, these implant components and/or implant surfaces are manufactured items, which have not contacted a bodily fluid and/or been inserted in a subject. Accordingly, such embodiments can be distinct from implant surfaces that have been worn from use within a patient's body.

The invention provides implants which have a surface with reduced susceptibility to wear (e.g., relative to an untreated implant surface, and/or a surface subjected to a selected amount of wear testing for a designated number of cycles, such as more than about 2 million cycles, and/or a surface with one or more quasi-steady-state surface characteristics with regard to wear). An implant include a substrate having a metal-on-substrate surface, the surface including a plurality of pits. The MOS surface can include a oxide-containing layer with a Cr:Co ratio greater than about 2 and a thickness greater than about 20 Å.

In some embodiments, the surface of a substrate can be configured as a MOS surface (e.g., a metallic surface). The MOS surface can have a plurality of pits, which can be configured (e.g., sized and/or numerous enough) to provide lubrication in the MOS interface. The plurality of pits can be defined as having a larger number density relative to a typical substrate surface such as a typical metallic surface, a typical chromium-containing alloy surface, a typical chromium-cobalt alloy surface, and/or a typical chromium-cobalt-molybdenum alloy surface. A typical surface can be of some standard substrate such as an ASTM standard substrate used in medical applications. In some instances, the pit density can be greater than a selected value such as about 10 pits/100 µm². In other instances, the pits can have an average size less than about 15 µm, or less than about 10 µm, or less than about 1 µm.

In some embodiments, the MOS surface can be inclusion-depleted (e.g., carbide-depleted) relative to a typical surface of the substrate which is coupled to the MOS surface. For example, the surface can have less inclusions than in a typical metallic surface, a typical chromium-containing alloy surface, a typical chromium-cobalt alloy surface, and/or a typical chromium-cobalt-molybdenum alloy surface. A typical surface can be defined by a particular ASTM standard of a metal (e.g., F75 or F1537). Accordingly, some embodiments can be drawn to a low carbon F1537 surface that can have less carbon inclusions than a high carbon F1537 metallic surface. In particular embodiments, an implant surface can be characterized by being substantially free, or relatively depleted with regard to an untreated substrate surface, of inclusions (e.g., carbides) having a selected size. For example, the surface can be substantially free, or depleted, of carbides and/or other inclusions having a size less than about 20 µm, less than about 15 µm, or less than about 10 µm, or less than about 1 µm.

In some embodiments, the surface (e.g., a pitted surface) can be characterized as having one or more portions that have low surface roughness. Low surface roughness can be characterized as being lower than the roughness of an etched surface using any of the techniques discussed herein on any of the substrates discussed herein. Low surface roughness can also be characterized by a Ra value below a selected value, such as below about 1 µm, about 0.6 µm, or about 0.1 µm.

In some embodiments, the surface has a ratio of components, which can be beneficial in reducing wear, for example by having an enhanced corrosion resistance to substances such as bodily fluids. For instance, the surface can have a high chromium content relative to other components. In some non-limiting examples, a surface can have a Cr:Co content above a selected ratio value, such as above about 1, 1.2, 1.5, 2, 2.5, or 3. In some instances, the ratio can have a value below about 20, 15, 10, 9, 8, or 7. Alternatively or in addition, the surface can have an oxide-containing layer, which can optionally have a selected thickness, such as greater than about 10Å, greater than about 15Å, or greater than about 20Å. A oxide-containing layer can also be characterized with a maximum thickness of about 200Å, 150Å, or 100Å.

Surfaces of substrates according to the invention can also be characterized by features which result from the methods of the invention. Accordingly, some embodiments are directed to implants having surfaces characterized by any portion or entirety of a surface treatment technique discussed herein. In one non-limiting example, an implant can include a treated surface, where the surface can be a portion of a metal-on-substrate surface. The treated surface can be produced by etching a portion of a chromium-cobalt alloy surface, which can include the formation of pits on the surface. The treated surface can also have a reduced inclusion content relative to the unetched chromium-cobalt surface. The etched surface can be smoothed, followed by creating an oxide-containing layer, which can be thick and/or have a thickness greater than a selected level on the surface, and modifying the oxide-containing layer to enhance the relative amount of at least one component of the layer. The techniques used to manufacture the surfaces can include additional steps.

### EXAMPLE 1: Treated CoCrMo Disk Surface with Chemical Etching

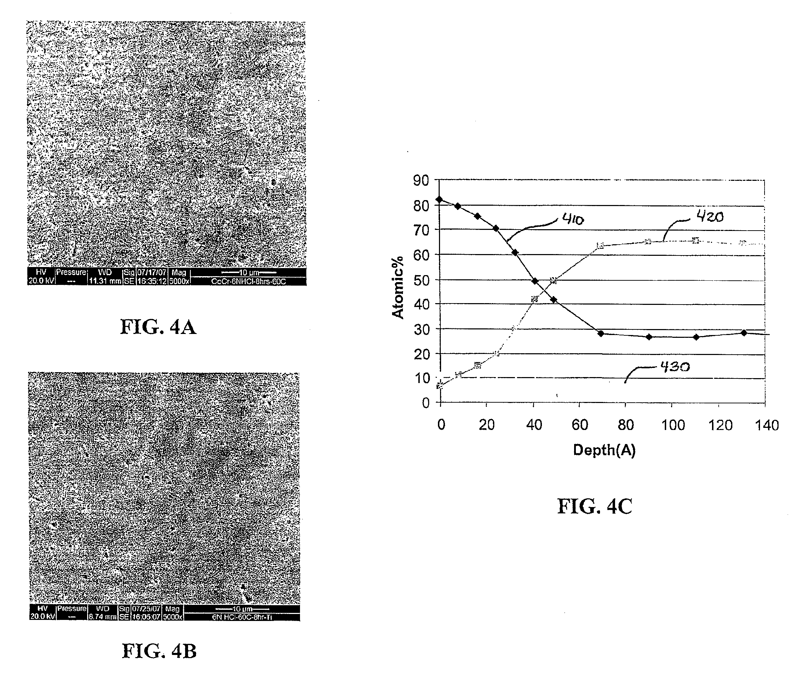

FIGS. 4A to 4C depict data associated with a treated cobalt-chromium-molybdenum disk. FIG. 4A shows a scanning electron micrograph (SEM) of a cobalt-chromium-molybdenum disk after chemical etching in 100 mL of 6 N HCl for 8 hours at 60°C. FIG. 4B shows the surface of the disk of FIG. 4A after the surface was polished with 0.1 µm alumina to an Ra value of about 0.1 µm, treated for about 1 hour with a plasma formed from molecular oxygen using a 1000 watt energy source, and subsequently exposed to a 6M HCl treatment for about an hour. FIG. 4C depicts a graph of the results of a XPS depth scan performed on the treated substrate of FIG. 4B. The traces 410, 420, 430 correspond with the atomic percentages as a function of depth of chromium, cobalt, and molybdenum, respectively. Accordingly, the graph of FIG. 4C shows that the chromium to cobalt ratio at the surface is about 13, and the oxide-containing layer of the surface is a little over 70Å thick.

### EXAMPLE 2: Treated CoCrMo Disk Surface with Electrochemical Etching

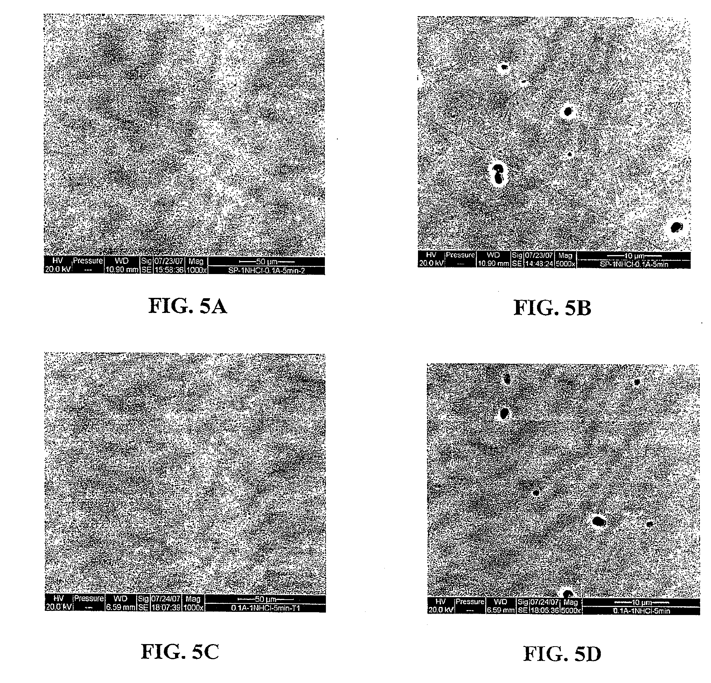

FIGS. 5A to 5F depict data associated with another treated cobalt-chromium-molybdenum disk. FIG. 5A shows an SEM of a cobalt-chromium-molybdenum disk after electrochemical etching in 300 mL of 1 N HCl for 5 minutes at room temperature. The disk served as the anode and a Pt electrode was used as a cathode. A current density of 32 mA.cm⁻² was utilized. FIG. 5B shows a section of the same surface as FIG. 5A but magnified about 5 times. The SEMs indicate that lower acid concentrations, lower temperatures, and shorter etching times, can be used effectively in electrochemical etching relative to the chemical etching of Example 1.

FIG. 5C shows the surface of FIG. 5A after the disk was mechanically polished with 0.1 µm alumina to a Ra value of about 0.1 µm. FIG. 5D shows a magnified view of a section of the surface depicted in FIG. 5C.

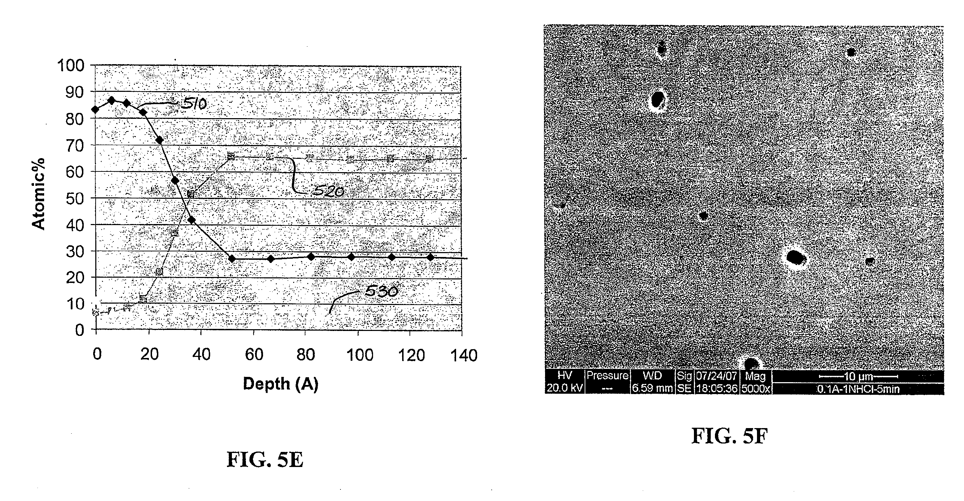

FIG. 5F shows the surface of the disk of FIG. 5C after the surface was treated for about 1 hour with a plasma formed from molecular oxygen using a 1000 watt energy source, and subsequently exposed to a 6M HCl treatment for about an hour. FIG. 5E depicts a graph of the results of a XPS depth scan performed on the treated substrate of FIG. 5F. The traces 510, 520, 530 correspond with the atomic percentages as a function of depth of chromium, cobalt, and molybdenum, respectively. Accordingly, the graph of FIG. 5E shows that the chromium to cobalt ratio at the surface is about 5, and the oxide-containing layer of the surface is about 50Å thick.

### EXAMPLE 3: Treated Femur Head

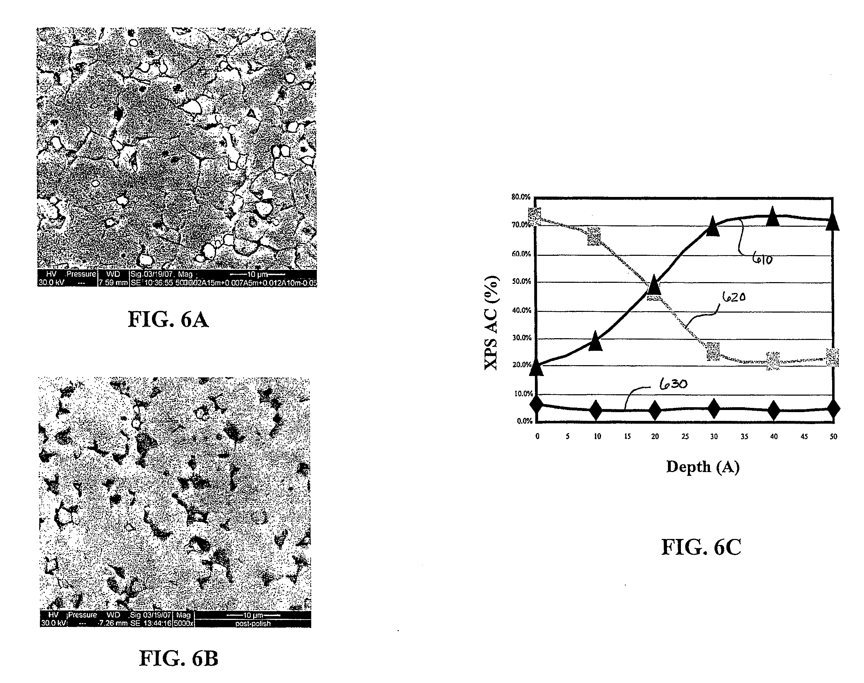

FIGS. 6A and 6B depict the treated surface of a chromium-cobalt-molybdenum femur head prosthesis. FIG. 6A shows a 5000X magnification SEM of an electrochemically etched femur head. FIG. 6B shows a 5000X magnification SEM of the same femur head after the surface was polished with 3/5 µm cloth to a Ra value of about 0.1 µm, treated with an oxygen plasma formed with a 1000 watt energy source for about one hour, and treated with nitric acid at 54°C for about half an hour. FIG. 6B shows that the polishing results in a substantially smoother surface. FIG. 6C is a plot of the results of a XPS depth scan performed on the treated femur head of FIG. 6B. The traces 610, 620, 630 correspond to the atomic percentages as a function of depth for cobalt, chromium, and molybdenum, respectively. The traces show that at the surface of the head, the chromium to cobalt ratio is a little over about 3.5.

## Claims

1. A method of treating an implant to reduce wear characteristics upon use of the implant in a subject, comprising:
forming a plurality of pits in at least a portion of a surface of the implant; and
forming a highly corrosion resistant oxide-containing layer on the pitted surface, the oxide-containing layer increasing the corrosion resistance of the pitted surface relative to a naturally-occurring oxide layer on the surface.

2. The method of claim 1, in which forming the plurality of pits comprises using a chemically-based etching technique, especially an electrochemical-based etching technique.

3. The method of claim 1, in which forming the plurality of pits involves removing a plurality of inclusions from the surface of the implant.

4. The method of claim 1, in which forming the oxide-containing layer involves treating the surface with a plasma having at least one oxidising component.

5. The method of claim 4, which includes the step of contacting the pitted surface with an acid after forming the oxide-containing layer.

6. The method of claim 4, in which the pitted surface comprises a cobalt-chromium alloy having a chromium to cobalt ratio greater than about 2.

7. The method of claim 1, in which forming the oxide-containing layer comprises forming the oxide-containing layer with a thickness greater than about 10Å.

8. The method of claim 1, in which forming the oxide-containing layer involves selectively enhancing a relative amount of at least one component in the oxide-containing layer.

9. The method of claim 8, in which forming the oxide-containing layer involves treating the oxide-containing layer with acid to selectively enhance the at least one component.

10. The method of claim 8, in which the implant comprises a cobalt-chromium alloy, and selectively enhancing the relative amount of at least one component comprises selectively enhancing chromium relative to cobalt in the oxide-containing layer, preferably so that the ratio of chromium to cobalt on the treated surface is in a range from about 2 to about 20.

11. The method of claim 1, which includes smoothing the pitted surface before forming the oxide-containing layer on the pitted surface.

12. The method of claim 11, in which smoothing the pitted surface comprises polishing the pitted surface.

13. The method of claim 11, in which at least a portion of the pitted surface is **characterized by** a Ra value less than about 0.6 µm.

14. The method of claim 1, in which the treated surface comprises at least a portion of a metal-on-substrate (MOS) surface of the implant.
